(19) Europäisches Patentamt
European Patent Office
Office européen des brevets

(11) Publication number: 0 138 374 B1

(12) **EUROPEAN PATENT SPECIFICATION**

(45) Date of publication of patent specification: 02.08.89

(51) Int. Cl.⁴: C 07 C 91/16, C 07 F 9/09, A 61 K 31/205, A 61 K 31/685

(21) Application number: 84306182.1

(22) Date of filing: 11.09.84

(54) **Salts of antimalarial phenanthrenemethanol compounds.**

(30) Priority: 16.09.83 US 533019
16.09.83 US 533018
02.11.83 US 548181

(43) Date of publication of application:
24.04.85 Bulletin 85/17

(45) Publication of the grant of the patent:
02.08.89 Bulletin 89/31

(84) Designated Contracting States:
AT BE CH DE FR GB IT LI LU NL SE

(56) References cited:
US-A-4 178 376
US-A-4 399 283

JOURNAL OF MEDICINAL CHEMISTRY, vol. 15, no. 7, 1972, pages 771-775, American Chemical Society, Easton, P.S., US; W.T. COLWELL et al.: "Antimalarial arylaminopropanols"

(73) Proprietor: SMITHKLINE BECKMAN CORPORATION
P.O. Box 7929 1 Franklin Plaza
Philadelphia Pennsylvania 19101 (US)

(72) Inventor: Rossignol, Jean Francois
8618 Germantown Avenue
Philadelphia Pennsylvania 19118 (US)

(74) Representative: Waters, David Martin, Dr. et al
Smith Kline & French Laboratories Ltd. Patent Department Mundells
Welwyn Garden City Hertfordshire AL7 1EY (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 138 374 B1

Courier Press, Leamington Spa, England.

**Description**

Background of the Invention

A number of phenanthrenemethanol compounds have been shown to exhibit antimalarial activity in humans against both chloroquine-sensitive and resistant strains of *Plasmodium falciparum.* The evaluation of the antimalarial activity of the phenanthrenemethanol, halofantrine or 1-[1,3-dichloro-6-trifluoromethyl-9-phenanthryl]-3-di-(*n*-butyl)-aminopropanol hydrochloride, was reported in the American Journal of Tropical Medicine and Hygiene, Vol. 31(6), pages 1075—79 (1982). Halofantrine was effective when administered over a short period of time and with a minimum of two doses against the multi-drug resistant Vietnam Smith strain and Cambodian Buchanan strain of *P. falciparum* and the Chesson strain of *P. vivax.* However, problems with systemic bioavailability remained. A means for enhancing the bioavailability of a number of phenanthrenemethanol antimalarial compounds, including halofantrine, utilizing specific organic fatty acids, as adjuvants, has been disclosed in U.S. Patent No. 4,178,376.

Summary of the Invention

This invention relates to salts of the class of antimalarial compounds containing halofantrine (as the free base) and its analogs, and more specifically the β-glycerophosphate, tartrate and biquinate salts. These salts exhibit markedly increased activity against malaria-causing parasites when compared to the hydrochloride salts reported in the literature. Pharmaceutical compositions and methods of treatment of subjects with malaria are also disclosed.

Detailed Description of the Invention

The compounds of this invention represented by the following structural formula (I):

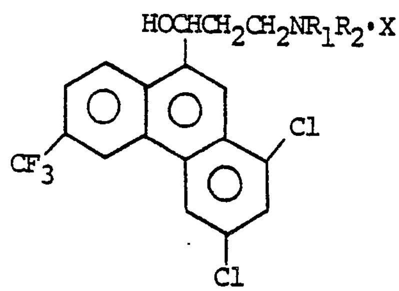

(I)

wherein $R_1$ is hydrogen or an alkyl radical containing one to six carbon atoms; $R_2$ is an alkyl radical containing one to six carbon atoms; and X is $H_2O_3POCH(CH_2OH)_2$, $n[HO_2CCH(OH)CH(OH)CO_2H]$ wherein n is 1/2 or 1, and $2HO_2CC_6H_7(OH)_4$, possess markedly increased activity against malaria-causing parasites.

A particular class of compounds of this invention are those compounds of formula (I) wherein $R_1$ is an alkyl radical containing one to six carbon atoms, and more particularly wherein both $R_1$ and $R_2$ are *n*-butyl radicals. Exemplifying this class of compounds are 1 - [1,3 - dichloro - 6 - trifluoromethyl - 9 - phenanthryl] - 3 - di - (*n* - butyl)aminopropanol - β - glycerophosphate, 1 - [1,3 - dichloro - 6 - trifluoromethyl - 9 - phenanthryl] - 3 - di - (*n* - butyl)aminopropanol - 1/2 - tartrate, 1 - [1,3 - dichloro - 6 - trifluoromethyl - 9 - phenanthryl] - 3 - d i - (*n* - butyl)aminopropanol tartrate, and 1 - [1,3 - dichloro - 6 - trifluoromethyl - 9 - phenanthryl] - 3 - di - (*n* - butyl)aminopropanol biquinate.

A second class of compounds of this invention are those compounds of the formula (I) wherein $R_1$ is hydrogen and $R_2$ is a *n*-butyl radical. Exemplifying this class of compounds is 1 - [1,3 - dichloro - 6 - trifluoromethyl - 9 - phenanthryl] - 3 - *n* - butylaminopropanol - β - glycerophosphate, 1 - [1,3 - dichloro - 6 - trifluoromethyl - 9 - phenanthryl] - 3 - *n* - butylaminopropanol - 1/2 - tartrate, 1 - [1,3 - dichloro - 6 - trifluoromethyl - 9 - phenanthryl] - 3 - *n* - butylaminopropanol tartrate, and 1 - [1,3 - dichloro - 6 - trifluoromethyl - 9 - phenanthryl] - 3 - *n* - butylaminopropanol biquinate.

The compounds of this invention are conveniently prepared by reacting β-glycerophosphoric acid, tartaric acid or quinic acid (1,3,4,5-tetrahydroxycyclohexanecarboxylic acid) with a compound of the formula (II):

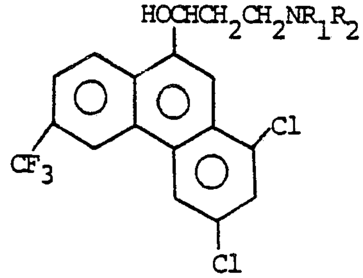

(II)

wherein $R_1$ and $R_2$ are described above in an inert solvent. The amount of β-glycerophosphoric acid employed in this reaction can be between 0.75 and 1.25 moles per mole of the compound of the formula (II) but equimolar amounts of both reactants are preferred. To prepare the tartrate salts of formula (I), wherein n is 1/2, the amount of tartaric acid employed in the reaction can be between 0.25 and 0.55 moles per mole of compound of the formula (II) but 0.50 moles of tartaric acid is preferred. Similarly, to prepare the tartrate salts of formula (I) wherein n is one, the amount of tartaric acid employed in the reaction can be between 0.75 and 1.25 moles per mole of the compound of formula (II) but equimolar amounts of both reactants are preferred. The amount of quinic acid employed in the above reaction can be between 1.50 and 2.50 moles per mole of the compound of the formula (II) but 2.0 moles of quinic acid is preferred.

The compounds of formula (II) is mixed with the appropriate amount of acid reactant (50% aqueous solution) and the inert solvent is added to affect solution of the reactants at a temperature selected from the range of ambient temperature to 100°C. The reaction solution is filtered and the filtrate is heated under reduced pressure up to 100°C to remove the solvent. Upon the removal of solvent, the respective compounds of the formula (I) precipitate and are collected and dried. The biquinate salts of formula (I) are partially water soluble to about 20 per cent on a weight by weight basis.

Examples of the inert solvents which are utilized in the process are alcohols, such as methanol, ethanol, isopropanol and the like and amides, such as dimethylformamide and dimethylacetamide.

The bases of the formula (II) are prepared according to the general procedures described in the Journal of Medicinal Chemistry, Vol. 15, No. 7, pages 771—5 (1972) wherein the process for converting substituted phenanthrene-9-carboxylic acid into the desired compounds as the free base form are detailed.

The antimalarial activities of the compounds of this invention is demonstrated in a standard pharmacological *in vivo* test procedure against *P. berghei* in Swiss mice.

The antimalarial activity of the compounds noted below was established utilizing the following methodology. Test animals were infected by interperitoneal injection of $10^6$ parasitized cells of *P. berghei* contained in 0.5 ml of 1:400 dilution of donor mouse blood which was infected with *P. berghei* one week earlier. Three days after infection, 3 groups of 4 mice (2 males and 2 females) were treated with the test compound at a dose level of 1, 4 and 16 mg/kg/day for 4 consecutive days. Similarly, a positive control of 3 groups of 4 mice (2 males and 2 females) were treated with halofantrine, as the hydrochloride salt, at a dose level of 3, 12 and 48 mg/kg/day for 4 consecutive days. The route of administration of the test compound and halofantrine was oral intubation of a suspension of each compound in 0.2% methyl celluose at a constant volume of 20 ml. A negative control group of 4 mice (2 males and 2 females) remained untreated. The results of the above test procedure (a) measured in survival time in days and percent of red cells parasitized at 28 days in the treated surviving mice and at 3 days in the untreated control mice or (b) expressed as an $ED_{50}$ (the effective dose which decreases the cell parasitemia by 50 percent on day 4 of treatment) are shown in the following Tables for the noted compounds.

Compound A: 1-[1,3-dichloro-6-trifluoromethyl-9-phenanthryl]-3-di-(*n*-butyl)aminopropanol-β-glycerophosphate;

Compound B: 1-[1,3-dichloro-6-trifluoromethyl-9-phenanthryl]-3-di-(*n*-butyl)aminopropanol-1/2-tartrate;

Compound C: 1-[1,3-dichloro-6-trifluoromethyl-9-phenanthryl]-3-di-(*n*-butyl)aminopropanol tartrate; and

Compound D: 1-[1,3-dichloro-6-trifluoromethyl-9-phenanthryl]-3-di-(*n*-butyl)aminopropanol biquinate.

TABLE I

| Dose mg/kg/day x 4 days | Deaths - % - Date of Death | % Red Cells Parasitized (Mean Value) |
|---|---|---|
| Halofantrine (HCl) | | |
| 3 mg/kg/day | 4/4 - 100% - day 5 | - |
| 12 mg/kg/day | 2/4 - 50% - day 8 and 13 | 16.5% |
| 48 mg/kg/day | 0/4 - 0% - | 3.0% |
| Compound A | | |
| 1 mg/kg/day | 4/4 - 100% - day 6 to 13 | - |
| 4 mg/kg/day | 0/4 - 0% - | 0% |
| 16 mg/kg/day | 0/4 - 0% - | 0% |
| Compound B | | |
| 1 mg/kg/day | 4/4 - 100% - day 5 to 7 | - |
| 4 mg/kg/day | 0/4 - 0% - | 0% |
| 16 mg/kg/day | 0/4 - 0% - | 0% |
| Compound C | | |
| 1 mg/kg/day | 3/4 - 75% - day 6 | 0% |
| 4 mg/kg/day | 0/4 - 0% - | 0% |
| 16 mg/kg/day | 0/4 - 0% - | 0% |
| Control | 4/4 - 100% - day 4 to 6 | 71.2% (day 3) |

TABLE II

| Dose mg/kg/day x 4 days | Number of Animals | $ED_{50}$ Day 4 (Mean Values) |
|---|---|---|
| Halofantrine (HCl) | | |
| 1 mg/kg/day | 4 | 6 mg/kg |
| 4 mg/kg/day | 4 | |
| 16 mg/kg/day | 4 | |
| Compound D | | |
| 1 mg/kg/day | 4 | ≤ 1 mg/kg |
| 4 mg/kg/day | 4 | |
| 16 mg/kg/day | 4 | |

Compounds A, B and C are at least 12 times more effective, on a weight basis, in the treatment of *P. berghei* malaria in mice based on survival times and clearance of parasites when compared to halofantrine hydrochloride.

Compound D is at least 6 times more effective, on a weight by weight basis, in the treatment of *P. berghei* malaria in mice using as an efficacy criterion the $ED_{50}$ calculation of deparasited cells on day 4 when compared to halofantrine hydrochloride. [See Annals of Tropical Medicine and Parasitology, *73*, pp. 505—525, (1979)].

The pharmaceutical compositions of this invention containing a compound of formula (I) which has antimalarial activity are prepared in conventinal dosage unit forms by incorporating the chemical compound with a nontoxic pharmaceutical carrier according to accepted procedures. A nontoxic quantity of said active ingredient is chosen which is sufficient to produce the desired chemotherapeutic activity in a subject, animal or human, without unacceptable toxicity. The compositions will contain the active ingredient in such an effective but nontoxic amount selected from about 125 mg to about 1000 mg of active ingredient per dosage unit but this quantity depends on the specific biological activity desired, the activity of the compound and the conditions of the patient.

A wide variety of pharmaceutical forms can be employed. Thus, if a solid carrier for oral administration is used the preparation can be tableted, placed in a hard gelatin capsule in powder or pellet form, or in the form of a suppository, trouche or lozenge. The amount of solid carrier will vary widely but preferably will be from about 125 mg to about 500 mg. If a liquid carrier is used, the preparation will be in the form of a syrup, emulsion, soft gelatin capsule, sterile injectable liquid such as an ampul, or an aqueous on nonaqueous liquid suspension.

The pharmaceutical preparations are made following the conventional technique of the pharmaceutical chemist involving mixing, granulating and compressing when necessary, or variously mixing and dissolving the ingredients as appropriate to give the desired end product.

The method of producing antimalarial activity, curatively or prophylactically, comprises administering internally to a subject in need of such activity a compound of formula (I), usually combined with a pharmaceutical carrier, in a nontoxic amount sufficient to produce said activity as described above. The route of administration may be any route which effectively transports the active compound to the site of action which is to be affected within the body such as orally or parenterally. Advantageously, a single oral dose or equal oral doses will be administered several times such as from 1—3 times a day with the daily dosage regimen being selected from about 125 mg to about 1000 mg.

The following examples illustrate the preparations of the compounds of formula (I) and their incorporation into pharmaceutical compositions.

## Example 1

Preparation of 1-[1,3-dichloro-6-trifluoromethyl-9-phenanthryl]-3-di-(*n*-butyl)aminopropanol-β-glycerophosphate (Compound A)

To a 50% aqueous solution of β-glycerophosphoric acid (4.8 g) at ambient temperature with stirring was added 1-[-1,3-dichloro-6-trifluoromethyl-9-phenanthryl]-3-di(*n*-butyl)aiminopropanol (14.0 g). To the mixture was added ethanol (500 ml) and the mixture is heated to about 100°C to affect solution. The solution is then filtered and the ethanol removed under vacuum until the desired product precipitates as a white crystalline material. After collecting and drying, the desired product has a melting point of 60—65°C. Elemental analysis is as follows: Calculated: C, 51.78; H, 5.80; N, 2.08 and Cl, 10.56. Found: C, 51.83; H, 5.65; N, 2.10 and Cl, 10.20.

## Example 2

Preparation of 1-[1,3-dichloro-6-trifluoromethyl-9-phenanthryl]-3-di-(*n*-butyl)aminopropanol-1/2-tartrate (Compound B)

To a 50% aqueous solution of tartaric acid (1.35 g) at ambient temperature with stirring was added 1-[-1,3-dichloro-6-trifluoromethyl-9-phenanthryl]-3-di(*n*-butyl)aminopropanol (90 g). To the mixture was added ethanol (500 ml) and the mixture is heated to about 100°C to affect solution. The solution is then filtered and the ethanol removed under vacuum until the desired product precipitates as a white crystalline material. After collecting and drying, the desired product has a melting point of 158°C. Elemental analysis is as follows: Calculated: C, 58.43; H, 5.77; N, 2.43 and Cl, 12.34 and F, 9.91. Found: C, 58.51; H, 5.40; N, 2.31; Cl, 11.99 and F, 9.70.

## Example 3

Preparation of 1-[1,3-dichloro-6-trifluoromethyl-9-phenanthryl]-3-di-(*n*-butyl)aminopropanol tartrate (Compound C)

Utilizing the general procedure of Example 2 with 2.7 tartaric acid and 9.0 g of 1-[1,3-dichloro-6-trifluoromethyl-9-phenanthryl]-3-di(*n*-butyl)aiminopropanol afforded the desired product as a white crystalline material with a melting point of 75—80°C. Elemental analysis is as follows: Calculated: C, 55.38; H, 5.53; N, 2.15 Cl, 10.92 and F, 8.77. Found: C, 54.92; H, 5.50; N, 2.00, Cl, 11.30 and F, 8.95.

Example 4

Preparation of 1-[1,3-dichloro-6-trifluoromethyl-9-phenanthryl]-3-di-(*n*-butyl)aminopropanol biquinate (Compound D)

To a 50% aqueous solution of quinic acid (4.62 g) at ambient temperature with stirring was added 1-[-1,3-dichloro-6-trifluoromethyl-9-phenanthryl]-3-di(*n*-butyl)aminopropanol (6 g). To the mixture was added ethanol (500 ml) and the mixture is heated to about 100°C to affect solution. The solution is then filtered and the ethanol and water removed under vacuum until dryness. The desired product solidifies as a white crystalline material with a melting point of about 90°C and is soluble in water up to 20 percent on a weight to weight basis. Elemental analysis is as follows: Calculated: C, 54.29; H, 6.32; N, 1.58, and Cl, 8.03, F, 6.44. Found: C, 54.32; H, 6.24; N, 1.66 and Cl, 7.95 F, 6.49.

Example 5

As specific embodiments of compositions of this invention, an active ingredient, such as one part of Compound A, B, C or D is dissolved in 20 parts of 0.2 percent aqueous methyl cellulose and is administered orally in one dose of 4 mg/kg to a subject in need of treatment of malaria.

**Claims for the Contracting States: BE FR DE IT LI LU NE SE CH UK**

1. A compound represented by the following structural formula (I):

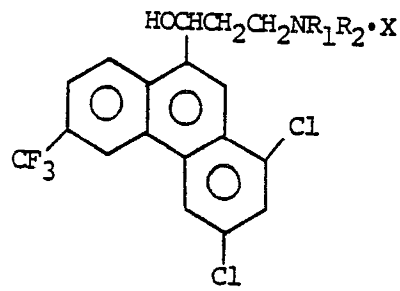

(I)

wherein $R_1$ is hydrogen or an alkyl radical containing one to six carbon atoms; $R_2$ is an alkyl radical containing one to six carbon atoms; and X is $H_2O_3POCH(CH_2OH)_2$, $n[HO_2CCH(OH)CH(OH)CO_2H]$ wherein n is 1/2 or 1, and $2HO_2CC_6H_7(OH)_4$.

2. A compound of claim 1 wherein $R_1$ and $R_2$ is an alkyl radical containing one to six carbon atoms.

3. A compound of claim 2 wherein both $R_1$ and $R_2$ are *n*-butyl radicals.

4. A compound of claim 3 which is 1 - [1,3 - dichloro - 6 - trifluoromethyl - 9 - phenanthryl] - 3 - di - (*n* - butyl)aminopropanol - β - glycerophosphate, 1 - [1,3 - dichloro - 6 - trifluoromethyl - 9 - phenanthryl] - 3 - di - (*n* - butyl)aminopropanol - hemitartrate, 1 - [1,3 - dichloro - 6 - trifluoromethyl - 9 - phenanthryl] - 3 - di - (*n*-butyl)aminopropanol monotartrate, or 1 - [1,3 - dichloro - 6 - trifluoromethyl - 9 - phenanthryl] - 3 - di - (*n* - butyl)aminopropanol biquinate.

5. A compound of claim 4 in a solid form.

6. A compound of claim 1 wherein $R_1$ is hydrogen.

7. A pharmaceutical composition comprising a compound as claimed in any one of claims 1 to 6 and a pharmaceutically acceptable carrier thereof.

8. A compound as claimed in any one of claims 1 to 6 for use as a therapeutic agent.

9. A compound as claimed in claim 8 for use as an antimalarial agent.

10. A process for the preparation of a compound as claimed in any one of claims 1 to 6 which comprises reacting the appropriate amount of β-glycerophosphoric acid, tartaric acid or quinic acid with a compound of the formula (II):

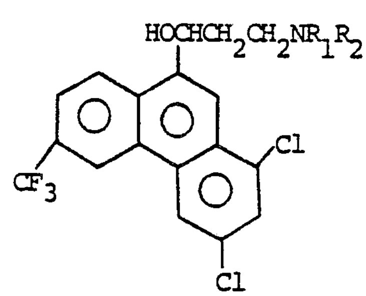

(II)

wherein $R_1$ and $R_2$ are as defined in claims 1, 2, 3 or 6 in an inert solvent.

**Claims for the Contracting State: AT**

1. A process for the preparation of a compound represented by the following structural formula (I):

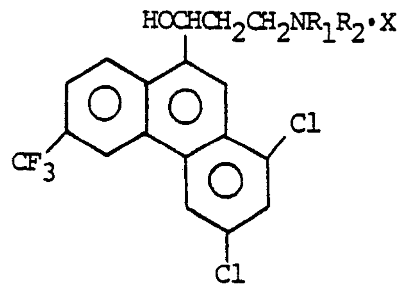

(I)

wherein $R_1$ is hydrogen or an alkyl radical containing one to six carbon atoms; $R_2$ is an alkyl radical containing one to six carbon atoms; and X is $H_2O_3POCH(CH_2OH)_2$, $n[HO_2CCH(OH)CH(OH)CO_2H]$ wherein n is 1/2 or 1, and $2HO_2CC_6H_7(OH)_4$, which comprises reacting β-glycerophosphoric acid, tartaric acid or quinic acid with a compound of the formula (II):

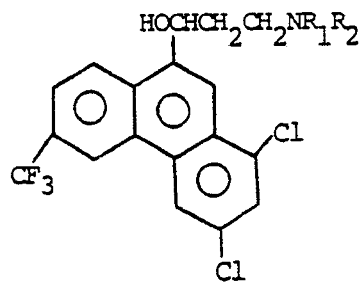

(II)

wherein $R_1$ and $R_2$ are as defined above, in an inert solvent.

2. The process of claim 1 wherein equimolar amounts of β-glycerophosphoric acid and a compound of the formula (II) are employed.

3. The process of claim 1 wherein equimolar amounts of tartaric acid and a compound of the formula (II) are employed.

4. The process of claim 1 wherein 0.50 moles of tartaric acid and 1.0 mole of a compound of the formula (II) are employed.

5. The process of claim 1 wherein 2.0 moles of quinic acid and 1.0 mole of a compound of the formula (II) are employed.

6. A process as claimed in any one of claims 1 to 5 in which the product is 1 - [1,3 - dichloro - 6 - trifluoromethyl - 9 - phenanthryl] - 3 - di - (*n* - butyl)aminopropanol - β - glycerophosphate, 1 - [1,3 - dichloro - 6 - trifluoromethyl - 9- p henanthryl] - 3 - di - (*n* - butyl)aminopropanolhemitartrate, 1 - [1,3 - dichloro - 6 - trifluoromethyl - 9 - phenanthryl] - 3 - di - (*n* - butyl)aminopropanol monotartrate, 1 - [1,3 - dichloro - 6 - trifluoromethyl - 9 - phenanthryl] - 3 - di - (*n* - butyl)aminopropanol biquinate, and the product is isolated as the solid particulate salt.

7. A process for the preparation of a pharmaceutical composition which comprises bringing into association a compound of structure (I) as defined in claim 1 and a pharmaceutically acceptable carrier therefor.

8. A process according to claim 7 in which the compound of structure (I) is 1 - [1,3 - dichloro - 6 - trifluoromethyl - 9 - phenanthryl] - 3 - di - (*n* - butyl)aminopropanol - β - glycerophosphate, 1 - [1,3 - dichloro - 6 - trifluoromethyl - 9 -phenanthryl] - 3 - di - (*n* - butyl)aminopropanolhemitartrate, 1 - [1,3 - dichloro - 6 - trifluoromethyl - 9 - phenanthryl] - 3 - di - (*n* - butyl)aminopropanol monotartrate, or 1 - [1,3 - dichloro - 6 - trifluoromethyl - 9 - phenanthryl] - 3 - di - (*n* - butyl)aminopropanol biquinate.

**Patentansprüche für die Vertragsstaaten: BE FR DE IT LI LU NL SE CH GB**

1. Eine Verbindung der folgenden Strukturformel (I)

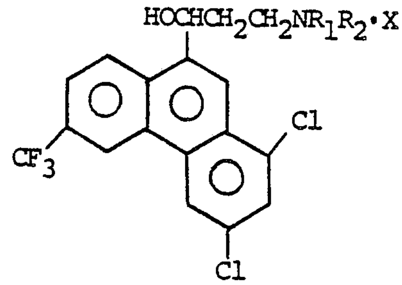

(I)

in der $R_1$ ein Wasserstoffatom oder einen Alkylrest mit 1 bis 6 Kohlenstoffatomen bedeutet, $R_2$ einen Alkylrest mit 1 bis 6 Kohlenstoffatomen darstellt und X $H_2O_3POCH(CH_2OH)_2$, $n[HO_2CCH(OH)CH(OH)CO_2H]$ wobei n 1/2 oder 1 ist, und $2HO_2CC_6H_7(OH_4$ ist.

2. Verbindung nach Anspruch 1, in der $R_1$ einen Alkylrest mit 1 bis 6 Kohlenstoffatomen bedeutet.

3. Verbindung nach Anspruch 2, in der sowohl $R_1$ als auch $R_2$ n-Butylgruppen sind.

4. Verbindung nach Anspruch 3, nämlich 1 - [1,3 - Dichlor - 6 - trifluormethyl - 9 - phenanthryl] - 3 - di(n - butyl) - aminopropanol - β - glycerophosphat, 1 - [1,3 - Dichlor - 6 - trifluormethyl - 9 - phenanthryl] - 3 - di(n - butyl) - aminopropanol - hemitartrat, 1 - [1,3 - Dichlor - 6 - trifluormethyl - 9 - phenanthryl] - 3 - di(n - butyl) - aminopropanol - monotartrat, oder 1 - [1,3 - Dichlor - 6 - trifluormethyl - 9 - phenanthryl] - 3 - di(n - butyl) - aminopropanol - di - chinasäure - salz.

5. Verbindung nach Anspruch 4 in fester Form.

6. Verbindung nach Anspruch 1, in der $R_1$ eine Wasserstoffatom ist.

7. Arzneimittel, umfassend eine Verbindung nach einem der Ansprüche 1 bis 6 und einen pharmazeutisch verträglichen Träger dafür.

8. Verbindung nach einem der Ansprüche 1 bis 6 zur Verwendung als therapeutischer Wirkstoff.

9. Verbindung nach Anspruch 8 zur Verwendung als Antimalariamittel.

10. Verfahren zur Herstellung einer Verbindung nach einem der Ansprüche 1 bis 6, das Umsetzung einer geeigneten Menge β-Glycerophosphorsäure, Weinsäure oder Chinasäure mit einer Verbindung der Formel (II)

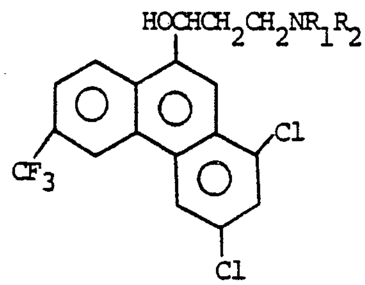

(II)

in der $R_1$ und $R_2$ wie in den Ansprüchen 1, 2, 3 oder 6 definiert sind, in einem inerten Lösungsmittel umfaßt.

**Patentansprüche für den Vertragsstaat: AT**

1. Verfahren zur Herstellung einer Verbindung der folgenden Strukturformel (I):

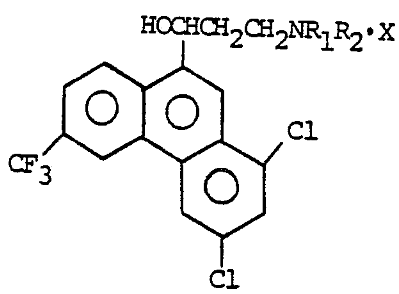

(I)

in der $R_1$ ein Wasserstoffatom oder einen Alkylrest mit 1 bis 6 Kohlenstoffatomen bedeutet, $R_2$ einen Alkylrest mit 1 bis 6 Kohlenstoffatomen darstellt und X $H_2O_3POCH(CH_2OH)_2$, $n[HO_2CCH(OH)CH(OH)CO_2H]$ wobei n 1/2 oder 1 ist, und $2HO_2CC_6H_7(OH)_4$ ist, welches Umsetzung von β-Glycerophosphorsäure,

Weinsäure oder Chinasäure mit einer Verbindung der Formel (II)

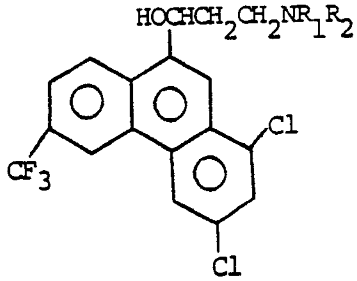

(II)

in der $R_1$ und $R_2$ wie vorstehend definiert sind, in einem inerten Lösungsmittel umfaßt.

2. Verfahren nach Anspruch 1, wobei äquimolare Mengen β-Glycerophosphorsäure und einer Verbindung der Formel (II) verwendet werden.

3. Verfahren nach Anspruch 1, wobei äquimolare Mengen Weinsäure und eine Verbindung der Formel (II) verwendet werden.

4. Verfahren nach Anspruch 1, wobei 0,5 Mol Weinsäure und 1,0 Mol einer Verbindung der Formel (II) verwendet werden.

5. Verfahren nach Anspruch 1, wobei 2,0 Mol Chinasäure 1,0 Mol einer Verbindung der Formel (II) verwendet werden.

6. Verfahren nach einem der Ansprüche 1 bis 5, wobei das Produkt 1-[1,3-Dichlor-6-trifluormethyl-9-phenanthryl]-3-di(n-butyl)-aminopropanol-β-glycerophosphat, 1-[1,3-Dichlor-6-trifluormethyl-9-phenanthryl]-3-di(n-butyl)-aminopropanol-hemitartrat, 1-[1,3-Dichlor-6-trifluormethyl-9-phenanthryl]-3-di(n-butyl)-aminopropanol-monotartrat, oder 1-[1,3-Dichlor-6-trifluormethyl-9-phenanthryl]-3-di(n-butyl)-aminopropanol-di-chinasäure-salz ist und das Produkt als festes, teilchenförmiges Salz gewonnen wird.

7. Verfahren zur Herstellung eines Arzneimittels, das Zusammenbringen einer Verbindung der Struktur (I) gemäß Anspruch 1 mit einem pharmarzeutisch verträglichen Träger dafür umfaßt.

8. Verfahren nach Anspruch 7, bei dem Verbindung der Struktur (I) 1-[1,3-Dichlor-6-trifluormethyl-9-phenanthryl]-3-di(n-butyl)-aminopropanol-β-glycerophosphat, 1-[1,3-Dichlor-6-trifluormethyl-9-phenanthryl]-3-di(n-butyl)-aminopropanol-hemitartrat, 1-[1,3-Dichlor-6-trifluormethyl-9-phenanthryl]-3-di(n-butyl)-aminopropanol-monotartrat, oder 1-[1,3-Dichlor-6-trifluormethyl-9-phenanthryl]-3-di(n-butyl)-aminopropanol-di-chinasäure-salz ist.

**Revendications pour les Etats contractants: BE FR DE IT LI LU NL SE CH GB**

1. Composé représente par la formule de structure générale suivante (I):

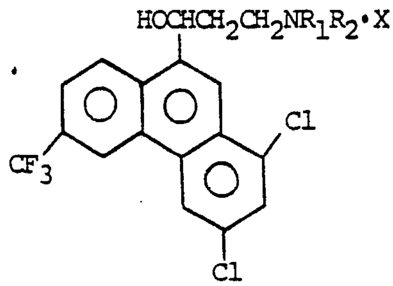

(I)

où $R_1$ est de l'hydrogène ou un radical alcoyle contenant de un à six atomes de carbone; $R_2$ est un radical alcoyle contenant de un à six atomes de carbone et X est $H_2O_3POCH(CH_2OH)_2$, $n[HO_2CCH(OH)CH(OH)CO_2H]$ où n est 1/2 et $2HO_2CC_6H_7(OH)_4$.

2. Composé de la revendication 1 où $R_1$ est un radical alcoyle contenant de un à six atomes de carbone.

3. Composé de la revendication 2 où $R_1$ et $R_2$ sont tous les deux un radical *n*-butyle.

4. Composé de la revendication 3 qui est le β-glycérophosphate de 1 - [1,3 - dichloro - 6 - trifluorométhyl - 9 - phénanthryl] - 3 - di(*n* - butyl)aminopropanol, l'hémitartrate de 1 - [1,3 - dichloro - 6 - trifluorométhyl - 9 - phénanthryl] - 3 - di(*n* - butyl)aminopropanol, le monotartrate de 1 - [1,3 - dichloro - 6 - trifluorométhyl - 9 - phénanthryl] - 3 - di(*n* - butyl)aminopropanol ou le bicinchonate de 1 - [1,3 - dichloro - 6 - trifluorométhyl - 9 - phénanthryl] - 3 - di(*n* - butyl)aminopropanol.

5. Composé de la revendication 4 sous forme solide.

6. Composé de la revendication 1 ou $R_1$ est de l'hydrogène.

7. Composition pharmaceutique comprenant un composé tel que revendiquè dans l'une quelconque des revendications 1 à 6 et un support pharmaceutiquement acceptable pour celui-ci.

8. Composé tel que revendiqué dans l'une quelconque des revendications 1 à 6 pour un usage comme agent thérapeutique.

9. Composé tel que revendiquè dans la revendication 8 pour un usage comme agent antimalarique.

10. Procédé pour la prépration d'un composé tel que revendiqué dans l'une quelconque des revendications 1 à 6 qui comprend la mise en réaction d'une quantité appropriée d'acide β-glycérophosphorique, d'acide tartrique ou d'acide cinchonique avec un composé de la formule (II):

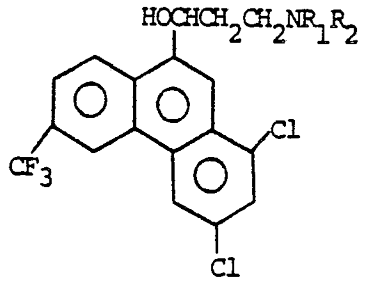

(II)

où $R_1$ et $R_2$ sont tels que définis dans la revendication 1, 2, 3 ou 6, dans un solvant inerte.

**Revendications pour l'Etat Contractant: AT**

1. Procédé pour la préparation d'un composé représententé par la formule de structure générale suivante (I):

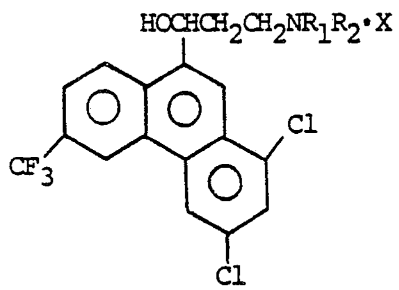

(I)

où $R_1$ est de l'hydrogène ou un radical alcoyle contenant de un à six atomes de carbone; $R_2$ est un radical alcoyle contenant de un à six atomes de carbone et X est $H_2O_3POCH(CH_2OH)_2$, $n[HO_2CCH(OH)CH(OH)CO_2H]$ où n et 1/2 ou 1 et $2HO_2CC_6H_7(OH)_4$, qui comprend la mise en réaction d'acide β-glycérophosphorique, d'acide tartrique ou d'acide cinchonique avec un composé de la formule (II)):

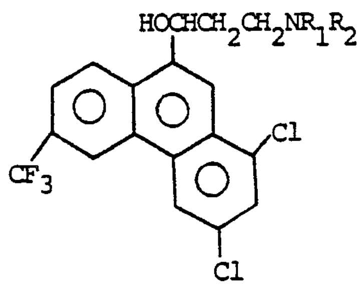

(II)

où $R_1$ et $R_2$ sont tels que définis plus haut, dans un solvant inerte.

2. Procédé de la revendication 1 dans lequel on emploie des quantités équimoléculaires d'acide β-glycérophosphorique et d'un composé de la formule (II).

3. Procédé de la revendication 1 dans lequel on emploie des quantités équimoléculaires d'acide tartrique et d'un composé de la formule (II).

4. Procédé de la revendication 1 dans lequel on emploie 0,5 mole d'acide tartrique et 1,0 mole d'un composé de la formule (II).

5. Procédé de la revendication 1 dans lequel on emploie 2,0 moles d'acide cinchonique et 1,0 mole d'un composé de la formule (II).

6. Procédé tel que revendiqué dans l'une quelconque des revendications 1 à 5 dans lequel le produit est le β - glycérophosphate de 1 - [1,3 - dichloro - 6 - trifluorométhyl - 9 - phénanthryl] - 3 - di(*n* - butyl)aminopropanol, l'hémitartrate de 1 - [1,3 - dichloro - 6 - trifluorométhyl - 9 - phénanthryl] - 3 - di(*n* - butyl)aminopropanol, le monotartrate de 1 - [1,3 - dichloro - 6 - trifluorométhyl - 9 - phénanthryl] - 3 - di(*n* - butyl)aminopropanol ou le bicinchonate de 1 - [1,3 - dichloro - 6 - trifluorométhyl - 9 - phénanthryl] - 3 - di(*n* - butyl)-aminopropanol et le produit est isolé sous la forme d'un sel solide particulaire.

7. Procédé pour la préparation d'une composition pharmaceutique qui comprend la mise en association d'un composé de formule (I) tel que défini dans la revendication 1 et un support pharmaceutiquement acceptable pour celui-ci.

8. Procédé suivant la revendication 7 dans lequel le composé de structure (I) est le β-glycérophosphate de 1-[1,3-dichloro-6-trifluorométhyl-9-phénanthryl]-3-di(*n*-butyl)aminopropanol, l'hémitartrate de 1-[1,3-dichloro-6-trifluorométhyl-9-phénanthryl]-3-di(*n*-butyl)aminopropanol, le monotartrate de 1-[1,3-dichloro-6-trifluorométhyl-9-phénanthryl]-3-di(*n*-butyl)aminopropanol ou le bicinchonate de 1-[1,3-dichloro-6-trifluorométhyl-9-phénanthryl]-3-di(*n*-butyl)aminopropanol.